# EUROPEAN PATENT APPLICATION

(11) **EP 4 831 146 A1**
(43) Date of publication of application: **28.10.2026**
(21) Application number: 26154243.5
(22) Date of filing: 27.01.2026
(51) Int. Cl.: C04B 35/488, C04B 35/486, A61C 13/083, A61K 6/818, C04B 35/645

(54) **ZIRCONIA SINTERED BODY AND MANUFACTURING METHOD THEREOF**

(30) Priority: 22.04.2025 KR 20250052297
(71) Applicant: Vatech mcis co., Ltd, Suwon-si, Gyeonggi-do 16648 (KR); Vatech Ewoo Holdings Co., Ltd., Hwaseong-si, Gyeonggi-do 18449 (KR)
(72) Inventor: HONG, Ju Seop, 16648 Suwon-si (KR); KIM, Jeong Jun, 16648 Suwon-si (KR); YANG, Gi Uk, 16648 Suwon-si (KR); CHAE, Ji Yeon, 16648 Suwon-si (KR)
(74) Representative: Lorenz & Kollegen

(57) **Abstract**

Provided are a zirconia sintered body and a manufacturing method thereof. The zirconia sintered body includes a powder for forming zirconia in a dispersed phase including a first raw material powder and a powder for forming zirconia in a matrix phase including a second raw material powder. The powder for forming zirconia in a dispersed phase and the powder for forming zirconia in a matrix phase form a mixed phase. The ratio of the matrix phase relative to the dispersed phase in the mixed phase ranges from 8:2 to 6:4.

## Description

### CROSS REFERENCE TO RELATED APPLICATION

The present application claims priority to Korean Patent Application No. 10-2025-0052297, filed April 22, 2025, the entire contents of which are incorporated herein for all purposes by this reference.

### BACKGROUND

### Technical Field

The disclosure relates to a zirconia sintered body and a manufacturing method thereof.

### Description of the Related Art

Zirconia, a dental ceramic material, is fully capable of replacing metal prosthetics and other ceramic materials in terms of strength and aesthetics. Zirconia is a material that is attracting attention across various industrial fields due to the chemical resistance, wear resistance, and high melting point thereof. Zirconia is also preferred as a biocompatible ceramic material because of being free from concerns regarding cytotoxicity, carcinogens, and heavy metals.

Zirconia has excellent mechanical properties, but a disadvantage that it is difficult to process a final sintered body due to high hardness. To address this problem, a porous zirconia semi-sintered body is first manufactured by primary sintering at a low temperature before completing the final sintered body. The porous zirconia semi-sintered body then undergoes a machining stage, followed by secondary final sintering to complete a prosthesis. Considering the shrinkage during the final sintering process, porous zirconia must be formed with a volume approximately 20% larger than the final size. However, semi-sintered zirconia has a problem that it is difficult to reduce the volume to match the actual size of the tooth of a patient during the final sintering process.

Due to the problems of durability, processing time, and machinability as described above, the production of prosthetics required significant time and expense. It was also difficult to process the final prosthetic into a structure optimally suited to the oral cavity of a patient, thereby limiting the competitiveness of zirconia prostheses.

Therefore, to reduce the fabrication time of prostheses, a technique has recently been proposed that shapes prostheses by directly machining zirconia sintered bodies in a partially sintered state, rather than machining zirconia calcined bodies.

However, zirconia sintered bodies have the problem of poor machinability due to high hardness. This poses a significant obstacle in the production of dental products from zirconia sintered bodies. Consequently, there is a need to develop sintered bodies that may improve strength as a final prosthetic material while also securing machinability sufficient to overcome issues such as tool breakage during the process of machining sintered bodies.

### SUMMARY

Accordingly, the disclosure has been made keeping in mind the above problems occurring in the related art, and the disclosure is intended to provide a zirconia sintered body including a mixture of dispersed and matrix phases and a manufacturing method thereof.

In order to achieve at least one of the above objectives, according to embodiments of the disclosure, there is provided a zirconia sintered body including: a powder for forming zirconia in a dispersed phase including a first raw material powder; and a powder for forming zirconia in a matrix phase including a second raw material powder, wherein the powder for forming zirconia in a dispersed phase and the powder for forming zirconia in a matrix phase form a mixed phase, the ratio of the matrix phase relative to the dispersed phase in the mixed phase ranging from 8:2 to 6:4.

According to an embodiment of the disclosure, the ratio of the matrix phase relative to the dispersed phase may be 7:3.

According to an embodiment of the disclosure, the matrix phase may have a grain size of 3 µm or greater, and the dispersed phase has a grain size less than 3 µm.

According to an embodiment of the disclosure, the weight ratio of the zirconia-forming raw material powder in a dispersed phase relative to the zirconia-forming raw material powder in a matrix phase may range from 1:2 to 1:10.

According to an embodiment of the disclosure, the first raw material powder may include 85 mol% to 95 mol% of ZrO₂, 3 mol% to 8 mol% of Y₂O₃, and 3 mol% to 8 mol% of Nb₂O₅ relative to a total amount of the first raw material powder.

According to an embodiment of the disclosure, the second raw material powder may include 80 mol% to 90 mol% of ZrO₂, 5 mol% to 10 mol% of Y₂O₃, and 5 mol% to 10 mol% of Nb₂O₅ relative to a total amount of the second raw material powder.

According to an embodiment of the disclosure, the powder for forming zirconia in a dispersed phase further may include a first sintering accelerator, the powder for forming zirconia in a matrix phase may further include a second sintering accelerator, the first sintering accelerator may be included at 0.1 wt% to 0.5 wt% relative to a total weight of the first raw material powder, and the second sintering accelerator may be included at 0.1 wt% to 0.5 wt% relative to a total weight of the second raw material powder.

According to an embodiment of the disclosure, the zirconia sintered body may further include an inorganic dye.

According to an embodiment of the disclosure, the inorganic dye may include at least one selected from a group consisting of Fe₂O₃, Er₂O₃, Co₃O₄, Mn₂O₃, and combinations thereof.

According to an embodiment of the disclosure, the inorganic dye may be included at 0.01 wt% to 10 wt% relative to a total weight of the zirconia sintered body.

According to embodiments of the disclosure, a method of manufacturing a zirconia sintered body includes: mixing a first raw material powder and a first sintering accelerator to produce a powder for forming zirconia in a dispersed phase; mixing a second raw material powder and a second sintering accelerator to produce a powder for forming zirconia in a matrix phase; mixing the powder for forming zirconia in a dispersed phase and the powder for forming zirconia in a matrix phase to produce a mixed powder; pressure molding the mixed powder to produce a molded body; and sintering the molded body.

Using the zirconia sintered body according to embodiments of the disclosure may ensure suitable machinability and maintain required strength during the manufacturing of dental products.

According to embodiments of the present disclosure, a zirconia sintered body with excellent crack resistance and fracture toughness may be provided.

The effects of the disclosure are not limited to the aforementioned effects, and other effects of the disclosure not explicitly described will be clearly understood from the description of the claims by a person having ordinary skill in the technical field, to which the disclosure pertains

### BRIEF DESCRIPTION OF THE DRAWINGS

The above and other objectives, features, and other advantages of the disclosure will be more clearly understood from the following detailed description when taken in conjunction with the accompanying drawings, in which:

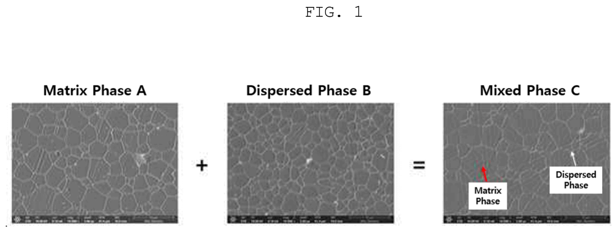

FIG. 1 is an image illustrating a microstructure of a zirconia sintered body according to an embodiment of the disclosure.

### DETAILED DESCRIPTION

Hereinafter, embodiments of the disclosure will be described in detail. However, these embodiments are presented merely as examples, by which the disclosure is not limited. The disclosure is defined solely by the scope of the claims set forth below.

Unless otherwise specified herein, when a portion such as a layer, a film, a region, or a plate is described as being "on" or "above" another portion, it should be understood that the portion may be "directly on" the other portion or indirectly on or above the other portion via an intervening portion.

Unless otherwise specified herein, singular forms may include plural forms. In addition, unless otherwise specified, "A or B" may refer to "including A, including B, or including A and B."

As used herein, the phrase "combination thereof" may refer to, for example, a mixture, a stack, a composite, a copolymer, an alloy, a blend, or a reaction product of constituents.

Methods and materials similar or equivalent to those described herein may be used in the practice or testing of the disclosure, but suitable methods and materials are described herein. Unless clearly stated otherwise in the context, singular forms include plural forms.

It will be understood herein that the terms "comprise", "include", "have", etc. when used in the specification, specify the presence of stated features, numbers, steps, operations, elements, parts, components, materials, and/or combinations thereof but do not preclude the presence or addition of one or more other features, numbers, steps, operations, elements, parts, components, materials, and/or combinations thereof.

As used herein, the term "a combination thereof" indicates a mixture or combination of two or more components described. The combination thereof may refer to, for example, a mixture, a stack, a composite, a copolymer, an alloy, a blend, or a reaction product of constituents.

As used herein, the term "and/or" refers to including any and all combinations of one or more of the items described in relation thereto. The term "or" in this specification refers to "and/or".

When a portion such as a layer, a film, a region, or a plate is described as being "on" or "above" another portion, it should be understood that the portion may be "directly on" the other portion or indirectly on or above the other portion via an intervening portion.

It will be understood that, although the terms "first", "second", etc., may be used herein to describe various elements, these elements should not be limited by these terms. These terms are only used to distinguish one element from another element.

Hereinafter, example embodiments will be described in more detail.

The zirconia sintered body according to embodiments of the disclosure includes a powder for forming zirconia in a dispersed phase containing a first raw material powder and a powder for forming zirconia in a matrix phase (also referred to as matrix zirconia) containing a second raw material powder.

The first raw material powder may be sintered to form zirconia in a dispersed phase (also referred to as dispersed zirconia). The dispersed zirconia may increase the strength and crack resistance of zirconia sintered body.

The first raw material powder may include 85 mol% to 95 mol% of ZrO₂, 3 mol% to 8 mol% of Y₂O₃, and 3 mol% to 8 mol% of Nb₂O₅ relative to the total amount of the first raw material powder.

The first raw material powder may include 87 mol% to 94 mol% of ZrO₂, 3 mol% to 6 mol% of Y₂O₃, and 3 mol% to 6 mol% of Nb₂O₅ relative to the total amount of the first raw material powder.

Because the first raw material powder contains ZrO₂, Y₂O₃, and Nb₂O₅ at the specified proportions, the zirconia sintered body may have excellent machinability and fracture toughness.

The second raw material powder may be sintered to form matrix zirconia. The matrix zirconia serves as the main component of a final zirconia sintered body, and may contribute to improving the machinability of the zirconia sintered body.

The second raw material powder may contain 80 mol% to 90 mol% of ZrO₂, 5 mol% to 10 mol% of Y₂O₃, and 5 mol% to 10 mol% of Nb₂O₅ relative to the total amount of the second raw material powder.

The second raw material powder may contain 82 mol% to 89 mol% of ZrO₂, 5.5 mol% to 9.5 mol% of Y₂O₃, and 5 mol% to 9 mol% of Nb₂O₅ relative to the total amount of the second raw material powder.

During the sintering process, the tetragonal phase of zirconia transforms into a monoclinic phase upon cooling at high temperatures, thereby causing approximately 3% volume expansion. This expansion may generate microcracks, which may degrade material properties. The presence of Y₂O₃ in the first raw powder may stabilize the zirconia sintered body to maintain the tetragonal phase even at room temperature.

When cracks form due to external forces, zirconia may undergo a localized phase transformation into a single-crystal structure. The resulting localized volume expansion may counteract the crack expanding force, thereby preventing crack propagation and improving resistance to defects. This phenomenon is referred to as zirconia toughening. Nb₂O₅ contained in the first raw material powder may further promote the toughness increasing mechanism.

Zirconia may experience low-temperature degradation, so that the tetragonal phase may transform into a monoclinic phase. This transformation may lead to microcracking due to overall volume expansion, thereby degrading the properties. Nb₂O₅ contained in the second raw material powder may suppress the degradation, thereby contributing to the production of stable #base powder at low temperatures.

The raw material powder for forming dispersed zirconia and the raw material powder for forming matrix zirconia may have a weight ratio of 1:2 to 1:10, 1:3 to 1:10, 1:3 to 1:9, or 1:4 to 1:9. Because the raw material powder for forming dispersed zirconia and the raw material powder for forming matrix zirconia are included at the above content ratios in a zirconia sintered body, the zirconia sintered body having excellent strength and processability may be produced.

The powder for forming dispersed zirconia and the powder for forming matrix zirconia may each be processed in a solid solution form.

The powder for forming dispersed zirconia may further include a first sintering accelerator, and the powder for forming matrix zirconia may further include a second sintering accelerator.

The first sintering accelerator and the second sintering accelerator may lower the sintering temperature or shorten the sintering time during the manufacturing process of zirconia sintered bodies, thereby improving the mechanical strength, wear resistance, and the like of the zirconia sintered bodies.

The first sintering accelerator and the second sintering accelerator may be at least one selected from the group consisting of, for example, TiO₂, Al₂O₃, CaO, MgO, NaCl, KCl, MgCl₂, SiO₂, B₂O₃, Sn, Ag, or combinations thereof.

The first sintering accelerator may be included at 0.1 wt% to 0.5 wt%, 0.1 wt% to 0.4 wt%, 0.15 wt% to 0.4 wt%, 0.15 wt% to 0.35 wt%, 0.2 wt% to 0.35 wt%, or 0.2 wt% to 0.3 wt% relative to the total weight of the first raw material powder.

The second sintering accelerator may be included at 0.1 wt% to 0.5 wt%, 0.1 wt% to 0.4 wt%, 0.15 wt% to 0.4 wt%, 0.15 wt% to 0.35 wt%, 0.2 wt% to 0.35 wt%, or 0.2 wt% to 0.3 wt% relative to the total weight of the second raw material powder.

Zirconia sintered body may further include an inorganic dye. The inorganic dye may serve to impart various colored hues to the final dental product manufactured. For example, the use of an inorganic dye may impart yellow, pink, or gray to a dental product.

The inorganic dye may include at least one selected from the group consisting of Fe₂O₃, Er₂O₃, Co₃O₄, Mn₂O₃, and combinations thereof.

Fe₂O₃ may contribute to the expression of yellow, Er₂O₃ to the expression of pink, and each of Co₃O₄ and Mn₂O₃ to the expression of gray. Co₃O₄, or Mn₂O₃ may be selectively used to impart gray to the final manufactured dental product.

The inorganic dye may be included at 0.01 wt% to 10 wt% relative to the total weight of the zirconia sintered body.

Fe₂O₃ may be included, for example, at 0.1 wt% to 1 wt%, 0.1 wt% to 0.9 wt%, or 0.1 wt% to 1 wt% relative to the total weight of the zirconia sintered body.

Er₂O₃ may be included, for example, at 0.1 wt% to 15 wt%, 0.1 wt% to 14 wt%, 0.1 wt% to 13 wt%, 0.1 wt% to 12 wt%, 0.1 wt% to 11 wt%, or 0.1 wt% to 10 wt% relative to the total weight of the zirconia sintered body.

Each of Co₃O₄ and Mn₂O₃ may be included, for example, at 0.1 wt% to 1 wt%, 0.1 wt% to 0.9 wt%, 0.1 wt% to 0.8 wt%, 0.1 wt% to 0.7 wt%, 0.1 wt% to 0.6 wt%, 0.1 wt% to 0.5 wt%, 0.1 wt% to 0.4 wt%, 0.1 wt% to 0.3 wt%, 0.1 wt% to 0.2 wt%, or 0.1 wt% to 0.1 wt% of the total weight of the zirconia sintered body.

As shown in FIG. 1, the zirconia sintered body may have a mixed-phase microstructure of matrix zirconia and dispersed zirconia. The ratio of the matrix phase relative to the dispersed phase in the mixed phase may range from 8:2 to 6:4.

The dispersed zirconia may have a grain size of 0.2 µm to 4 µm, 0.4 µm to 4 µm, 0.5 µm to 4 µm, 0.7 µm to 4 µm, 0.7 µm to 3.5 µm, 0.7 µm to 3 µm, 1 µm to 3 µm, or 1 µm to 2.5 µm. Desirably, the dispersed zirconia may have a grain size of less than 3 µm.

The matrix zirconia may have a grain size of 1 µm to 10 µm, 1.5 µm to 10 µm, 2 µm to 10 µm, 2 µm to 9.5 µm, 2 µm to 8 µm, 2.5 µm to 8 µm, or 3 µm to 8 µm. Desirably, the matrix zirconia may have a grain size of 3 µm or greater.

Due to the mixed-phase microstructure of the matrix zirconia and the dispersed zirconia, the zirconia sintered body may have not only excellent mechanical properties but also improved machinability.

In the microstructure of the zirconia sintered body, the matrix phase may be provided at a ratio greater than 50% of the total mixed phase. Specifically, the matrix phase, which has a larger grain size (crystal grain size) than the dispersed phase, may be provided at a greater proportion than the dispersed phase. That is, it is desirable that the proportion of the matrix phase is greater than 50% of the total mixed phase, and that the proportion of the dispersed phase is less than 50% of the total mixed phase.

In an embodiment, matrix phase particles having a grain size of 3 µm or more may be provided in a greater number than dispersed phase particles having a grain size of less than 3 µm. Desirably, the ratio of the matrix phase relative to the dispersed phase may be 7:3, and in other examples, may be 9:1 or in the range of 8:2 to 6:4.

The ratio of the matrix phase relative to the dispersed phase in the mixed phase of the zirconia sintered body may be determined by distinguishing the matrix phase and the dispersed phase based on the aforementioned grain size observed in the mixed phase using a scanning electron microscope (SEM). Then, the ratio of the matrix phase relative to the dispersed phase may be specified according to the number of distinguished matrix and dispersed phases. For example, in a case in which a mixed phase observed at a specified magnification has 7 grains equal to or larger than 3 µm and 3 grains smaller than 3 µm, the ratio of the matrix phases relative to the dispersed phases in the mixed phase may be specified as 7:3.

Based on the grain sizes and ratio of the matrix phase relative to the dispersed phase as described above, it is possible to achieve the strength required for dental prostheses while also ensuring machinability during machining processes such as milling.

The strength of the zirconia sintered body may be 300 MPa to 900 MPa, 350 MPa to 900 MPa, 350 MPa to 850 MPa, 350 MPa to 800 MPa, 350 MPa to 750 MPa, or 400 MPa to 750 MPa.

The hardness of the zirconia sintered body may be 5 GPa to 12 GPa, 5.5 GPa to 12 GPa, 5.5 GPa to 11.5 GPa, 5.5 GPa to 11 GPa, 6 GPa to 11 GPa, 6.5 GPa to 11 GPa, 7 GPa to 11 GPa, 7 GPa to 10.5 GPa, or 7 GPa to 10 MPa.

The fracture toughness of the zirconia sintered body may be 7 MPa ·m^{1/2} to 16 MPa ·m^{1/2}, 7.5 MPa ·m^{1/2} to 16 MPa ·m^{1/2}, 8 MPa ·m^{1/2} to 16 MPa ·m^{1/2}, 8 MPa ·m^{1/2} to 15.5 MPa ·m^{1/2}, 8 MPa ·m^{1/2} to 15 MPa ·m^{1/2}, 8.5 MPa ·m^{1/2} to 15 MPa ·m^{1/2}, 9 MPa ·m^{1/2} to 15 MPa ·m^{1/2}, 9 MPa ·m^{1/2} to 14.5 MPa ·m^{1/2}, or 9 MPa ·m^{1/2} to 14 MPa ·m^{1/2}.

A manufacturing method of a zirconia sintered body according to embodiments of the disclosure includes: mixing a first raw material powder and a first sintering accelerator to produce a powder for forming zirconia in a dispersed phase (also referred to as dispersed zirconia); mixing a second raw material powder and a second sintering accelerator to produce a powder for forming zirconia in a matrix phase (also referred to as matrix zirconia); mixing the powder for forming dispersed zirconia and the powder for forming matrix zirconia to produce a mixed powder; pressure molding the mixed powder to produce a molded body; and sintering the molded body.

The same as described above for the zirconia sintered body may apply to the first raw material powder, the second raw material powder, the first sintering accelerator, the second sintering accelerator, the powder for forming dispersed zirconia, and the powder for forming matrix zirconia.

In the step of manufacturing the mixed powder, the powder for forming dispersed zirconia and the raw material powder for forming matrix zirconia may be mixed at a weight ratio of 1:2 to 1:10, 1:3 to 1:10, 1:3 to 1:9, or 1:4 to 1:9.

Dry pressure molding may be performed during the step of manufacturing the molded body. The pressure molding may be performed within a pressure range of 100 to 300 MPa.

The step of sintering the molded body may include pre-sintering and full sintering.

The pre-sintering may be performed within a temperature range of 600°C to 1400°C. Performing the pre-sintering may prevent cracks from forming during the full sintering and create bonding force.

The full sintering may be performed within a temperature range of 1200°C to 1800°C.

After the full sintering, pressure sintering may be further performed to enhance the mechanical and optical properties of the sintered body. The pressure sintering may be performed under conditions of 800 bar to 2500 bar and 1000°C to 2000°C.

For example, a zirconia sintered body manufactured according to embodiments has the following composition and properties.

**Table 1**

| Classification | | Dispersed Phase (10 to 20 wt%) | Matrix Phase (80 to 90 wt%) |
|---|---|---|---|
| Function of Microstructure | | Improve Strength | Ensure Block Machinability |
| Grain Size | | 1.0 to 2.5 µm | 3.0 to 8.0 µm |
| Composition Contents | ZrO2 | 88.8 to 93.0 mol% | 82.3 to 88.3 mol% |
| | Y203 | 3.7 to 5.9 mol% | 6.0 to 9.0 mol% |
| | Nb205 | 3.3 to 5.3 mol% | 5.7 to 8.7 mol% |

**Table 2**

| Strength (MPa) | Hardness (GPa) | Fracture Toughness (MPa ·m1/2) |
|---|---|---|
| 400 to 750 | 7 to 10 | 9 to 14 |

A dental product manufactured using a zirconia sintered body according to embodiments of the disclosure may be provided. The dental product may include dental prostheses, orthodontic products, or dental implant products. Dental prostheses may include, for example, crowns, dental crowns, dentures, and the like. Orthodontic products may include, for example, orthodontic brackets. Dental implant products may include, for example, dental implant crowns.

Although the disclosure has been described in detail with reference to the example embodiments, the disclosure is not limited to these embodiments. Various modifications are possible within the scope of the claims, the detailed description of the disclosure, and the accompanying drawings, and such modifications should also fall within the scope of the disclosure.

## Claims

1. A zirconia sintered body comprising:
a powder for forming zirconia in a dispersed phase comprising a first raw material powder; and
a powder for forming zirconia in a matrix phase comprising a second raw material powder,
**characterized in that** the powder for forming zirconia in a dispersed phase and the powder for forming zirconia in a matrix phase form a mixed phase within a sintered body,
the ratio of the matrix phase relative to the dispersed phase in the mixed phase ranging from 8:2 to 6:4.

2. The zirconia sintered body of claim 1, wherein the ratio of the matrix phase relative to the dispersed phase is 7:3.

3. The zirconia sintered body of claim 1, wherein the matrix phase has a grain size of 3 µm or greater, and the dispersed phase has a grain size less than 3 pm.

4. The zirconia sintered body of claim 1, wherein the weight ratio of the zirconia-forming raw material powder in a dispersed phase relative to the zirconia-forming raw material powder in a matrix phase ranges from 1:2 to 1:10.

5. The zirconia sintered body of claim 4, wherein the first raw material powder comprises 85 mol% to 95 mol% of ZrO₂, 3 mol% to 8 mol% of Y₂O₃, and 3 mol% to 8 mol% of Nb₂O₅ relative to a total amount of the first raw material powder.

6. The zirconia sintered body of claim 4, wherein the second raw material powder comprises 80 mol% to 90 mol% of ZrO₂, 5 mol% to 10 mol% of Y₂O₃, and 5 mol% to 10 mol% of Nb₂O₅ relative to a total amount of the second raw material powder.

7. The zirconia sintered body of claim 4, wherein the powder for forming zirconia in a dispersed phase further comprises a first sintering accelerator,
the powder for forming zirconia in a matrix phase further comprises a second sintering accelerator,
the first sintering accelerator is included at 0.1 wt% to 0.5 wt% relative to a total weight of the first raw material powder, and
the second sintering accelerator is included at 0.1 wt% to 0.5 wt% relative to a total weight of the second raw material powder.

8. The zirconia sintered body of claim 4, further comprising an inorganic dye.

9. The zirconia sintered body of claim 8, wherein the inorganic dye comprises at least one selected from a group consisting of Fe₂O₃, Er₂O₃, Co₃O₄, Mn₂O₃, and combinations thereof.

10. The zirconia sintered body of claim 8, wherein the inorganic dye is included at 0.01 wt% to 10 wt% relative to a total weight of the zirconia sintered body.

11. A method of manufacturing a zirconia sintered body, the method comprising:
mixing a first raw material powder and a first sintering accelerator to produce a powder for forming zirconia in a dispersed phase;
mixing a second raw material powder and a second sintering accelerator to produce a powder for forming zirconia in a matrix phase;
mixing the powder for forming zirconia in a dispersed phase and the powder for forming zirconia in a matrix phase to produce a mixed powder;
pressure molding the mixed powder to produce a molded body; and
sintering the molded body.
